# EUROPEAN PATENT APPLICATION

(11) **EP 1 127 875 A1**
(43) Date of publication of application: **29.08.2001**
(21) Application number: 00103665.6
(22) Date of filing: 22.02.2000
(51) Int. Cl.: C07D 209/20

(54) **Process for the preparation of alpha-dimethylaminomethyl-tryptophan methyl ester**

(71) Applicant: WARNER-LAMBERT COMPANY, Morris Plains New Jersey 07950 (US)
(72) Inventor: Barth, Hubert, Dr., 79312 Emmendingen (DE); Steiner, Klaus, Dr., 79312 Emmendingen (DE); Schiefermayr, Bernd, 79112 Freiburg-Opfingen (DE)
(74) Representative: Tesch, Rudolf, Dr.

(57) **Abstract**

The invention is concerned with a process for the preparation of α-dimethylaminomethyltryptophan methyl ester, wherein (S)-2-benzylidene-amino)-3-(1H-indol-3-yl)-propionic acid methyl ester is reacted with 1-dimethylaminomethylbenzotriazole to give racemic α-dimethylaminomethyltryptophan methyl ester.

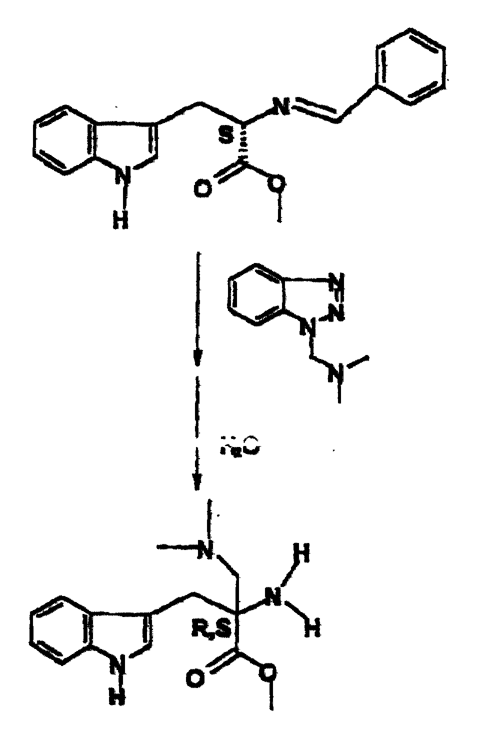

## Description

The present invention is concerned with a simplified process for the preparation of α-dimethyl-aminomethyltryptophan methyl ester (2-amino-3-(1H-indol-3-yl)-2-dimethylaminomethylpropionic acid methyl ester) and of its enantiomers.

α-Dimethylaminomethyltryptophan methyl ester is a key compound for the preparation of a new class of highly effective NK1 receptor antagonists.

These NK1 receptor antagonists are diastereomerically pure compounds which hitherto could only be prepared by a 10 - 11 step synthesis.

As a rule, the main part of these NK1 receptor antagonists is an (S)-α-dimethylaminomethyltryptophan component. Since hitherto it was not possible directly to prepare racemic α-dimethylaminomethyltryptophan methyl ester or its (S)- and (R)-enantiomers, the synthesis had to take place via laboriously protected tryptophan derivatives.

For this purpose, Boc-tryptophan was first converted into the corresponding allyl ester. In the next reaction step, the indolic nitrogen atom had to be protected by reaction with triisopropylsilyl chloride. After splitting off the Boc protective group, the amino ester obtained was converted by means of benzaldehyde into the corresponding imino compound (Schiff's base). This Schiff's base was aminoalkylated in the α-position in the presence of a strong base (hexamethyldisilazane lithium salt) and dimethyl-methylene ammonium iodide (Eschenmoser's salt). During the aqueous working up, the imino group functioning as protective group was again split off to give an α-aminoalkylated racemic tryptophan derivative protected not only on the indole nitrogen but also on the carbonyl atom, this tryptophan derivative having the formula:

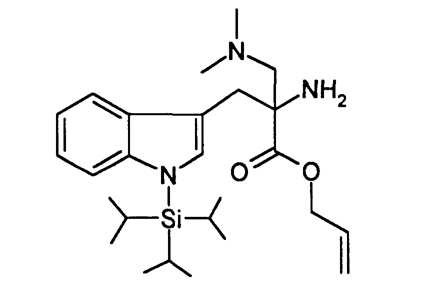

In three further reaction steps, including one with an optically pure amine, this protected tryptophan derivative was reacted to give a diastereomeric mixture. Only at the stage of the tryptophan derivative still protected on the indole nitrogen it was possible to separate the two diastereomeric compounds by means of chromatographic methods. After splitting off the triisopropylsilyl protective group, the diastereomeric pure final compound was obtained.

In the case of the previously used process of preparation, the following problems arose.

1. as starting material, it was necessary to use the expensive Boc-tryptophan (Boc-Trp-OH);

2. the acid function of the Boc-Trp-OH used as starting material had to be converted into the allyl ester in a laborious synthesis;

3. in order to prevent a subsequent alkylation of the indole nitrogen atom of the tryptophan derivative, this must be blocked by the introduction of a triisopropylsilyl protective group;

4. after splitting off the Boc protective group, the resulting amine must be converted into an imine by means of benzaldehyde;

5. the α-aminoalkylation with the expensive and sensitive Eschenmoser's salt only takes place with low and variable yields;

6. a protected enantiomeric mixture results which cannot be separated into its enantiomers;

7. the allyl and triisopropyl protective groups can first be split off, when in the course of the further synthesis, a diastereomeric mixture is formed and this has been separated by chromatographic methods;

8. due to the laborious protective group chemistry and separation of the diastereomers only possible late in the synthesis, a high loss of materials results and, consequently, an increase of the synthesis costs;

9. furthermore, it was, surprisingly, ascertained that it is not possible to convert α-dimethylaminomethyltryptophan prepared in other ways into the α-dimethylaminomethyltryptophan methyl ester by conventional methods of esterification.

NK1 receptor antagonists have been developed which contain an (S)-α-dimethylaminomethyltryptophan or (R)-α-dimethylaminomethyltryptophan component.

Starting from Boc-Trp-OH, the NK1 receptor antagonists can, in part, be prepared by a laborious at least 10 step synthesis. During the synthesis, a racemic α-dimethylaminomethyltryptophan derivative protected on the indole nitrogen atom must hereby be built up which derivative must be reacted with a further chiral component to give a diastereomeric mixture. A separation of the protected racemic α-dimethylaminomethyltryptophan derivative was not possible. Only at the stage of the diastereomeric mixture was a separation into the two diastereomeric compounds possible by means of chromatographic methods. In a subsequent reaction, the introduced protective groups, which are essential for the separation of the diastereomeric mixture, must again be split off. This laborious and expensive synthesis is not suitable for the large scale preparation of the above-mentioned NK1 receptor antagonists.

Based on experiences with previous syntheses with α-methyltryptophan methyl ester, the use of α-dimethylaminomethyltryptophan methyl ester appeared to be desirable.

Consequently, ways were sought to prepare the desired α-dimethylaminomethyltryptophan methyl ester without the use of laborious and expensive protection group chemistry contrary to literature references, the desired selective α-aminoalkylation with 1-dimethylaminomethylbenzotriazole, starting from the Schiff's base ((S)-2-benzylideneamino)-3-(1H-indol-3-yl)-propionic acid methyl ester), could be achieved.

The racemic α-dimethylaminomethyltryptophan methyl ester can then be further reacted in the usual way with a second chiral component to give a diastereomeric mixture which can be separated by crystallisation into the two diastereomeric compounds.

By the use of unprotected racemic α-dimethylaminomethyltryptophan methyl ester, the number of synthesis steps could be reduced from 11 to 5. However, a disadvantage was the late separation of the diasteromeric mixture, resulting in loss of material.

Attempts to separate the racemic α-dimethylaminomethyltryptophan methyl ester by enzymatic methods or after formation of diastereomeric salts into the two enantiomers were unsuccessful. However, initially it was possible to separate the two enantiomeric compounds on an analytical scale on a chiral HPLC phase.

However, a chiral phase was tested which is found to be suitable for the separation of racemic α-dimethylaminomethyltryptophan methyl ester by means of preparative HPLC into the two enantiomers.

Thus, it is an object of the present invention to provide an economic process which can be carried out on a large scale for the preparation of the above-mentioned key compound, i.e. α-dimethylaminomethyltryptophan methyl ester, without thereby having to use protective groups or Eschenmoser's salt.

Surprisingly, we have now found that (S)-2-benzylidene-amino)-3-(1H-indol-3-yl)-propionic acid methyl ester (Schiffs base), which can be prepared in one synthesis step from the cheap (S)-tryptophan methyl ester ((S)-Trp-OMe), can, by reaction with 1-dimethylaminomethylbenzotriazole, be converted into racemic α-dimethylaminomethyltryptophan methyl ester.

It is known that N-(α-aminoalkyl)-benzotriazole derivatives, which can be prepared very easily from benzotriazole, an aldehyde and a primary or secondary amine, can be used as aminoalkylation reagents (see A. Katritzky et al., Tetrahedron, 46, No. 24, 8153-8160/1990).

The preparation of 1-dimethylaminomethylbenzotriazole has been described by J.H. Bruckhalter et al. (J.A.C.S., 74, 3868-3369/1952).

B.E. Love and B.T. Nguyeri (Synlett, 1123, October, 1998) have described the reaction of 1-methylaminomethylbenzotriazole and indole. As main reaction, there hereby takes place an aminoalkylation on the indole nitrogen atom, an aminoalkylation on the 3-position only taking place as a secondary reaction.

Surprisingly, in the case of the tryptophan derivative (Schiffs base) used in the case of the present invention, this aminoalkylation reaction with 1-dimethylaminomethyl-benzotriazole only takes place on the α-C atom. In contradistinction to the above-mentioned literature references, an alkylation on the indole nitrogen atom was not observed.

Furthermore, we have found that the racemic α-dimethylaminomethyltryptophan methyl ester cannot be separated into the enantiomers either enzymatically or after formation of diastereomeric salts. It was even more surprising that the two enantiomers, i.e. the (S)-enantiomer and the (R)-enantiomer, can be separated on a preparative scale on a chiral phase.

It was also very surprising that α-dimethylaminomethyltryptophan can only be esterified in conventional manner with extreme difficulty.

The reaction sequence according to the present invention is shown in the following scheme:

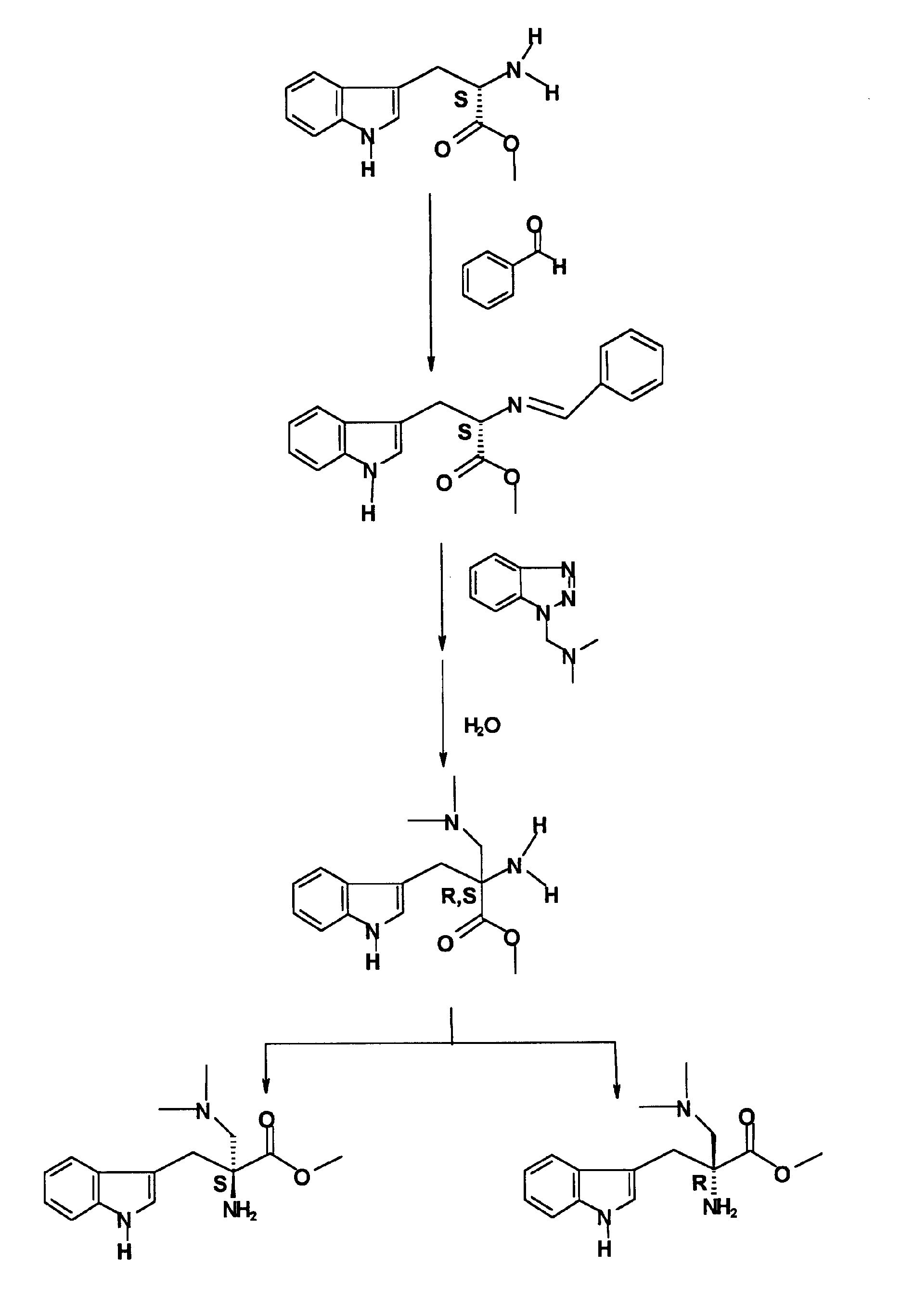

The following Examples are given for the illustration of the present invention:

### Example 1

Preparation of (S)-2-(benzylidene-amino)-3-(1H-indol-3-yl)-propionic acid methyl ester (Schiffs base).

245 g (S)-Tryptophan methyl ester and 118 g benzaldehyde are dissolved in 1837 ml dichloromethane and mixed with 245 g dry magnesium sulphate. The reaction mixture is stirred for 4 hours at ambient temperature. After filtering off the magnesium sulphate, the solvent is distilled off on a rotary evaporator. The very viscous mass remaining behind (364 g) can be used directly in the next step.

### Example 2

Preparation of racemic α-dimethylaminomethyltryptophan methyl ester

117.9 g Diisopropylamine are dissolved in 1170 ml anhydrous tetrahydrofuran in a reaction flask gassed with nitrogen with the exclusion of moisture. 728 ml 20 of a 1.6 M butyl lithium/hexane solution are added dropwise at -30°C within the course of about 1 hour. After stirring for about 15 minutes, 340 g of the Schiff's base prepared in Example 1, dissolved in 1020 ml anhydrous tetrahydrofuran, are added dropwise at -30°C over the course of 1 hour. After a further 15 minutes, 205 g 1-dimethylaniinomethyl-benzotriazole, dissolved in 1025 ml anhydrous tetrahydrofuran are added dropwise at -30°C over the course of 1 hour. Subsequently, the reaction mixture is allowed to warm up slowly to ambient temperature. During the following approximately 16 hours stirring at ambient temperature, a thick slurry results.

With ice cooling, a solution of 386 ml 37% hydrochloric acid in 1544 ml ice water is allowed to run in in such a manner that the temperature in the reaction vessel does not exceed 25°C. The separating tetrahydrofuran phase is separated off and the aqueous phase extracted five times with 500 ml amounts of ethyl acetate.

The aqueous phase is covered with 500 ml ethyl acetate and mixed portionwise, while stirring, with 247 g sodium carbonate. The organic phase is separated off and the aqueous phase again extracted twice with, in each case, 500 ml ethyl acetate. The combined organic phases are washed twice with, in each case, 300 ml of a saturated aqueous solution of sodium chloride.

After drying over anhydrous sodium sulphate, the solution was filtered clear and evaporated in a vacuum.

There remain 242 g (79.1 % of theory) of a brown, viscous residue. According to HPLC analysis, this crude product contains about 62% of the desired product. For further purification, the crude product is recrystallised from diethyl ether to give 94 g (30.7% of theory) racemic α-dimethylaminomethyltryptophan methyl ester; m.p. 105°'C; HPLC purity 96.2 rel.%.

### Example 3

Separation of racemic α-dimethylaminomethyltryptophan methyl ester into the two enantiomeric compounds.

15 g of the racemic α-dimethylaminomethyltryptophan methyl ester obtained in Example 2 are separated on a preparative HPLC apparatus on the chiral phase Chiracel OJ 20 µm into 6.2 g of the (R)-enantiomer and 6.45 g of the (S)-enantiomer.

## Claims

1. Process for the preparation of α-dimethylaminomethyltryptophan methyl ester, wherein (S)-2-benzylidene-amino)-3-(1 H-indol-3-yl)-propionic acid methyl ester is reacted with 1-dimethylaminomethylbenzotriazole to give racemic α-dimethylaminomethyltryptophan methyl ester.

2. Process according to claim 1, wherein the (S)-2-benzylideneamino)-3-(1 H-indol-3-yl)-propionic acid methyl ester is prepared from (S)-tryptophan methyl ester by reaction with benzaldehyde.

3. Process according to claim 1 or 2, wherein the racemic methyl ester obtained is separated into the (R)- and (S)-enantiomers by preparative HPLC or by crystallisation.
